# EUROPEAN PATENT SPECIFICATION

(11) **EP 2 747 681 B1**
(45) Date of publication and mention of the grant of the patent: **30.08.2017**
(21) Application number: 12838960.8
(22) Date of filing: 05.10.2012
(51) Int. Cl.: A61N 2/06

(54) **DYNAMIC MULTI-LAYER THERAPEUTIC MAGNETIC DEVICE**
MEHRSCHICHTIGE MAGNETVORRICHTUNG FÜR DYNAMISCHE THERAPIEN
DISPOSITIF MAGNÉTIQUE THÉRAPEUTIQUE MULTICOUCHES DYNAMIQUE

(30) Priority: 07.10.2011 US 201161545013 P
(43) Date of publication of application: 02.07.2014
(73) Proprietor: Nikken International, Inc., Irvine, CA 92618 (US)
(72) Inventor: BALZER, David, Irvine, California 92618 (US); MOON, Sungwook, Irvine, California 92618 (US)
(74) Representative: Prüfer & Partner mbB Patentanwälte · Rechtsanwälte
(86) International application number: PCT/US2012/059110
(87) International publication number: WO 2013/052904

(56) References cited:
- CN-A- 101 485 506
- KR-B1- 100 941 369
- US-A- 5 233 768
- US-A1- 2004 060 651
- US-A1- 2004 230 139
- US-A1- 2006 075 781
- US-A1- 2009 216 068

## Description

### CROSS-REFERENCES TO RELATED APPLICATIONS

This patent application claims the benefit of U.S. Patent document 2016166845 published on 16-06-2016, which document is incorporated herein by this reference thereto.

### BACKGROUND OF THE INVENTION

This invention relates to the field of magnetic therapy devices, and in particular to the field of consumer magnetic therapy devices that produce a multitude of magnetic flux lines at the application surface.

Document US 2004/230139 A1 describes a floating massage pad structure comprising a pair of magnetic members, one disposed in a massage protrusion and another disposed in an opposing member, wherein the pair of magnetic members are configured to repel the massage protrusion and the opposing member. Document US 2004/060651 A1 describes a method for fabricating a magnetic therapeutic device comprising permanent magnets that are magnetically attracted by each other.

### SUMMARY OF THE INVENTION

The invention relates to a device for therapeutic use on a mammal according to claim 1, and to a method for forming a device for use on a mammal according to claim 7. The invention is a multi-layer magnetic device comprising two or more layers of containing magnets or ferromagnetic material to be applied to areas of the body of a mammal in which the first layer has one or more protrusions and the second layer has one or more receiving zones that are positioned to align with the protrusions of the first layer. At least a portion of the first layer has a first magnetic pattern, and at least a portion of the second layer has a second magnetic pattern such that, when the protrusions are aligned with the receiving zones, at least one region of the first magnetic pattern faces at least one region of the second magnetic pattern of like polarity thereby repulsing one another.

### BRIEF DESCRIPTION OF THE DRAWINGS

Figure 1 is a top plan view of one embodiment of a therapeutic magnetic device in keeping with the present invention.
Figure 2 is a cross-sectional view of the device of Figure 1 taken along centerline thereof showing the protrusions **106** of the first member **100** aligned with the receiving zones **206** of the second member **200** in keeping with one embodiment of the present invention.
Figure 3 is a cross-sectional view of a device similar to that of Figure 6, except that the device additionally comprises a sheet **302** between the first and second members in keeping with another embodiment of the present invention.
Figure 4 is a top plan view of the first member **100** of the device of Figure 1.
Figure 5 is a cross-sectional view of the first member **100** of Figure 4 taken along centerline thereof.
Figure 6 is a top plan view of the second member **200** of the device of Figure 1.
Figure 7 is a cross-sectional view of the second member **200** of Figure 6 taken along centerline thereof.
Figure 8 is a top plan view of another first member **100** in keeping with one embodiment of the present invention wherein the protrusions **106** and the receiving zones **206** are a combination of triangle and diamond shapes.
Figure 9 is a cross-sectional view of the first member **100** of Figure 8 taken along centerline thereof.
Figure 10 is a top plan view of a second member **200** configured to align with the first member **100** of Figure 8.
Figure 11 is a cross-sectional view of the second member **200** of Figure 10 taken along centerline thereof.
Figure 12 is a top plan view of another first member **100** in keeping with one embodiment of the present invention wherein the protrusions **106** and the receiving zones **206** are a combination of circumferential and radial shapes.
Figure 13 is a cross-sectional view of the first member **100** of Figure 12 taken along centerline thereof.
Figure 14 is a top plan view of a second member **200** configured to align with the first member **100** of Figure 12.
Figure 15 is a cross-sectional view of the second member **200** of Figure 14 taken along centerline thereof.
Figure 16 is a top plan view of another first member **100** in keeping with one embodiment of the present invention wherein the receiving zones **206** are recessions that do not continue through the entire thickness of the second member **200** and, as just one example of such an embodiment, form a checkerboard of recessions for aligning with a matching checkerboard of protrusions **106** on the mating surface **102** of the first member **100.**
Figure 17 is a cross-sectional view of the first member **100** of Figure 16 taken along centerline thereof.
Figure 18 is a top plan view of a second member **200** configured to align with the first member **100** of Figure 16.
Figure 19 is a cross-sectional view of the second member **200** of Figure 18 taken along centerline thereof.
Figure 20 is a top plan view of another first member **100** in keeping with one embodiment of the present invention wherein the magnetic pattern imposed thereon is non-homogeneous.
Figure 21 is a cross-sectional view of the first member **100** of Figure 20 taken along centerline thereof.
Figure 22 is a top plan view of a second member **200** configured to align with the first member **100** of Figure 20.
Figure 23 is a cross-sectional view of the second member **200** of Figure 22 taken along centerline thereof.
Figure 24 is a top plan view of another first member **100** in keeping with one embodiment of the present invention wherein the magnetic pattern imposed thereon is a different non-homogeneous pattern.
Figure 25 is a cross-sectional view of the first member **100** of Figure 24 taken along centerline thereof.
Figure 26 is a top plan view of a second member **200** configured to align with the first member **100** of Figure 24.
Figure 27 is a cross-sectional view of the second member **200** of Figure 26 taken along centerline thereof.
Figure 28 is a top plan view of another first member **100** in keeping with another embodiment of the present invention wherein there are more than two separate layers.
Figure 29 is a cross-sectional view of the first member **100** of Figure 28 taken along centerline thereof.
Figure 30 is a top plan view of a second member **200** configured to align with the first member **100** of Figure 28 and further having protrusions to align with further members.
Figure 31 is a cross-sectional view of the second member **200** of Figure 30 taken along centerline thereof.
Figure 32 is a top plan view of a third member **300** configured to align with the first member **100** of Figure 28 and/or the second member **200** of Figure 30.
Figure 33 is a cross-sectional view of the third member **300** of Figure 32 taken along centerline thereof.

### DESCRIPTION OF THE PREFERRED EMBODIMENTS

The detailed description set forth below in connection with the appended drawings is intended as a description of presently-preferred embodiments of the invention and is not intended to represent the only forms in which the present invention may be constructed and/or utilized. The description sets forth the functions and the sequence of steps for constructing and operating the invention in connection with the illustrated embodiments. However, it is to be understood that the same or equivalent functions and sequences may be accomplished by different embodiments that are also intended to be encompassed within the scope of the invention as defined by the claims.

One embodiment of the present invention comprises two (2) separate and distinct layers of flexible, ferromagnetic material combined into a small planar material to be applied to areas of the body. The first layer is the "cast" layer in which a number of protrusions are formed on one side; the second layer is the "die-cut" layer having a number of holes that match the arrangement of protrusions of the cast layer. Before the layers are aligned and assembled, each layer is separately magnetized. The two layers are then assembled with either the two south poles or the two north poles facing each other. Once the layers are aligned and pressed together, the protrusions from the cast layer and the application surface of the die-cut layer form a surface magnetic pattern that has both polarities.

One of the key fabrication advantages of this approach is that the magnetization of each layer can be induced after casting or stamping but before pressing the two layers together. Another key fabrication advantage of this approach is that it is readily amenable to a virtually unlimited number of protrusion and receiving zone variations in the choice of number, size, shape, and proximity of such protrusions. Thus, a variety of flux line configurations may be produced simply by varying the number, size, and placement of the protrusions and receiving zones.

One of the key functional advantages in some embodiments of the present invention is that the forced like-pole juxtaposition of members **100** and **200** may produce magnetic fields that extend considerably deeper into the user's body. Also, the placement and configurations of the protrusions and recesses themselves can be fashioned to increase the overall depth of penetration once applied to the surface of the body. A further key functional advantage is that the repulsive forces between two layers once assembled will act to form a magnetic flux pattern that is dynamic, increasing and decreasing as the user compresses and then releases the device while in use.

For example, using this magnetic configuration in shoe insoles, as the user applies his/her weight to the insole, the weight forces the layers together, closing the air gap created by the magnetic repulsion between the two layers, thereby resulting in a fluid-type flux "pumping."

### Example 1

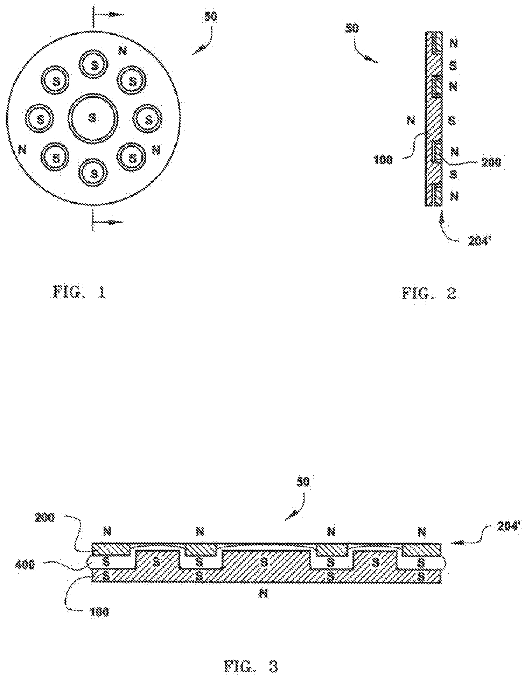

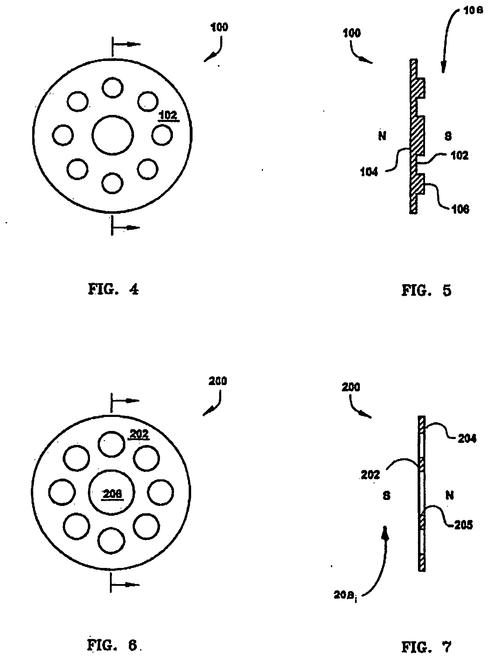

Thus, as shown in Figures 1 through 7, one embodiment of the present invention involves a therapeutic magnetic device **50** for use on a human or an animal comprising a first member **100** and a second member **200.** The first member **100,** which may be a thin, flexible sheet material, comprises a mating surface **102** and distal surface **104.** The mating surface **102** of the first member **100** comprises a number of protrusions **106.**

The second member **200,** which likewise may be a thin, flexible sheet material, comprises a mating surface **202** and a distal surface **204.** The mating surface **202** of the second member **200** has one or more internal edges **205** that define a plurality of receiving zones **206** for aligning with the plurality of protrusions **106.** The receiving zones **206** may be orifices or may be recesses, depending on the intended application and other dimensional parameters of the first and second members **100** and **200** and the desired magnetic field gradients at the application surface.

The first member **100,** or at least a portion of it, is induced with a magnetic pattern **108.** The second member **200,** or at least a portion of it, is similarly induced with a magnetic patterns **208** such that, when the protrusions **106** are aligned with the receiving zones **206,** at least a portion of the first magnetic pattern **108** faces at least a portion of the second magnetic pattern **208** in such a manner as to have like polarities facing each other thereby producing a repulsive magnetic force on one another.

The result is a therapeutically effective plurality of magnetic flux lines at an application surface **204'** of the device **50,** which in some embodiments, is distal surface **204** of the second member **200.** It is also intended, in some embodiments, that a number of characteristics of this plurality of magnetic flux lines will vary as the distance L between the mating surface **102** of the first member **100** and mating surface **202** of the second member **200** varies.

One benefit of some embodiments of this innovation is that the magnetization processes are greatly simplified. The first member **100** with its plurality of protrusions **106** may be placed in a magnetic field of a desired magnetic pattern **108** so as to impose this magnetic pattern **108** upon the protrusion-bearing mating surface **102.** The second member **200** with its plurality of receiving zones **206** may be placed in a separate magnetic field of a desired magnetic pattern **208** so as to impose this separate magnetic pattern **208** upon the mating surface **202** of the second member **200** with its receiving zones **206.** Then, by mating the plurality of protrusions **106** on the first sheet with the plurality of receiving features **206** on the second sheet, portions of the two magnetic patterns **108** and **208** come into repulsive magnetic communication with one another.

Another benefit of other embodiments of the present invention is that the magnetic patterns and interactions created by the aligning layers produce at an application surface a therapeutically beneficial plurality of magnetic flux lines in multiple directions in a predetermined pattern. And, in some embodiments, this plurality of magnetic flux lines is dynamic, varying in strength, and sometimes even in direction and depth, as the layers are compressed despite the repulsive forces acting between the layers.

The first member **100** may be forced into alignment with the second member **200** but the supporting structures on either side of the first and second members **100** and **200.** Although not shown in Figures 1 and 2, first member **100** may be supported by a larger structure, such as a casing, an insole for insertion into a shoe, or a sheet or other cloth portion of a blanket or the like (not shown in the Figures). Second member **200,** likewise, may be supported by a larger structure, such as a connected portion of the casing, a second layer of an insole, or a second sheet or cloth that comprises a blanket. In such embodiments, first member **100** is forced up against the second member **200.**

Once the two members **100** and **200** are aligned as discussed above and forced against one another by such larger structure, the two facing magnetic patterns repel each other forcing the first and second members **100** and **200** apart, whether greatly or only slightly. In either case, the dynamic nature of the plurality of magnetic flux lines created by these repulsive forces may be therapeutically significant at the application surface and deep within the tissues of the user.

Alternatively, as illustrated in Figures 1 and 2, the first member **100** may be press fit onto the second member **200,** once the two members are aligned as discussed above, or they may be connected by an adhesive or the like such that first member **100** may still move relative to second member **200** but still remain connected to, or at least aligned with and in magnetic communication with, the second member **200.** Further alternatively, as shown in Figure 3, the first and second members **100** and **200** may be separated from one another by using an elastic sheet that may further facilitate or enhance the ability of first member **100** to move relative to second member **200** during use.

The protrusion/receiving zone combinations in some embodiments can act as a plurality of protruding keying features on the first sheet with a plurality of receiving keying features on the second sheet. The mating of these features then act as a key and keyhole locking in the intended orientation of the first member **100** relative to the second member **200,** causing at least one region of the first magnetic pattern **108** to face at least one region of the second magnetic pattern **208** of like polarity in repelling fashion. As the distance between the first and second sheets varies, the therapeutically effective plurality of magnetic flux lines produced thereby at an application surface of the device **50** will vary.

In some embodiments, the first and second members **100** and **200** may be separated by a non-magnetic material **400,** such as an elastomer, adhesive, or the like, as shown in Figure 3. This non-magnetic material **400** may be used to help maintain the two members **100** and **200** in proper relationship, reduce wear and tear, and/or maintain members **100** and **200** at a predetermined range of distance between one another in order to modulate the magnetic flux lines at the application surface.

### Example 2

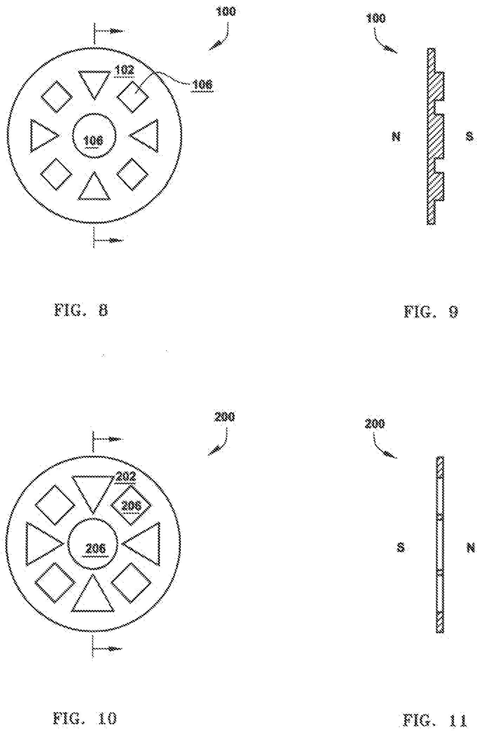

Figures 8 through 11 show another embodiment of the present invention wherein the protrusions **106** and the receiving zones **206** are a combination of triangle and diamond shapes. Additionally, although the device **50** of Figures 1 through 7 and 8 through 11 are illustrated as circular, the device **50** could just as easily be configured into any shape. In fact, triangular, square, and hexagonal shaped devices could better cover a two-dimensional region, such as a pad, blanket, insole, or the like. Such other shapes are fully and equally contemplated as alternative embodiments within the present invention.

### Example 3

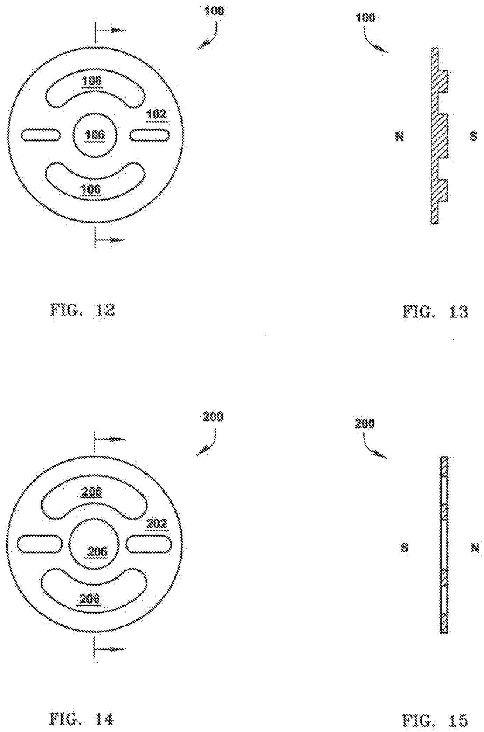

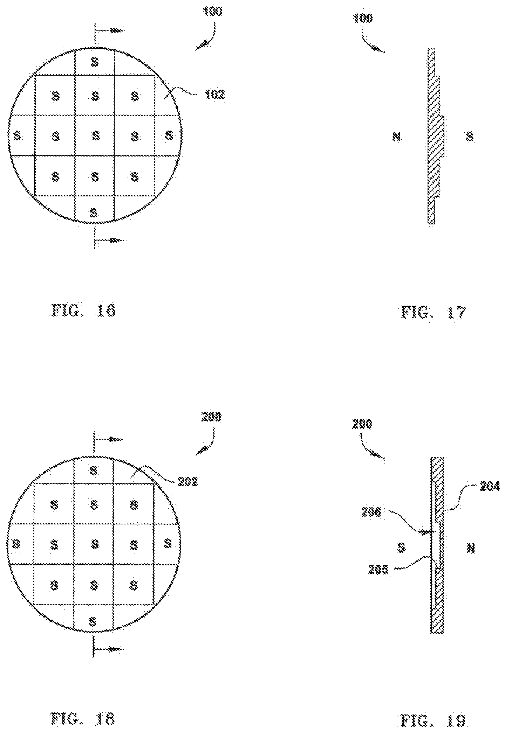

Similarly, shown in Figures 12 through 15 is another embodiment of the present invention wherein the protrusions **106** and the receiving zones **206** are a combination of circumferential and radial shapes, and shown in Figures 16 through 19, is still a further embodiment of the present invention wherein the receiving zones **206** are recessions that do not continue through the entire thickness of the second member **200** and, as just one example of such an embodiment, form a checkerboard of recessions for aligning with a matching checkerboard of protrusions **106** on the mating surface **102** of the first member **100.**

### Examples 4 and 5

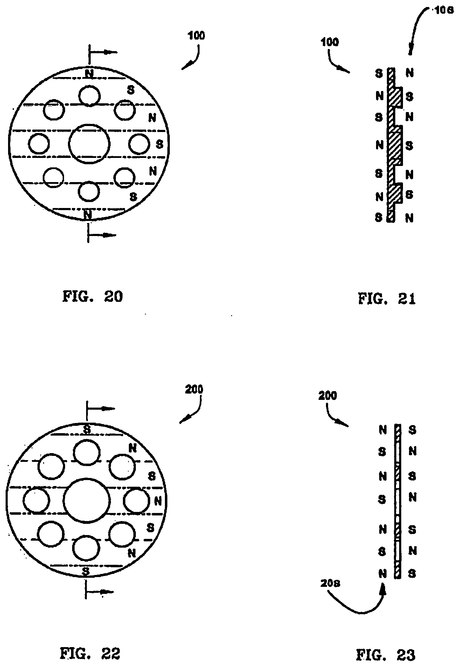

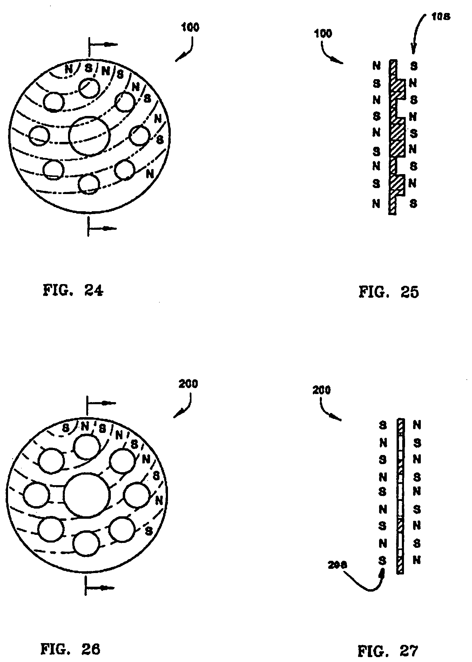

Although the members **100** and **200** of the embodiments discussed so far have been illustrated as having generally homogeneous magnetic patterns, as shown in Figures 20 through 28, other embodiments of the present invention may include any number of other non-homogeneous patterns imposed on the first and second members **100** and **200,** provided that at least some portion of the magnetic pattern **108** on the first member encounters a portion of the magnetic pattern **208** on the second member **200** in such a way that a repulsive magnetic force is exerted between the two layers.

### Example 6

In some embodiments, there may be further members or layers, in which the second member **200,** or even the first member **100,** may similarly align with the additional layer or layers that exhibit similarly opposing magnetic patterns **108** and **208,** each layer comprising protrusions and/or receiving zones that may facilitate the above-mentioned alignment and engage in magnetic communication with other layers so as to product a repulsive force and/or a plurality of magnetic flux lines at an application surface of the device **50.**

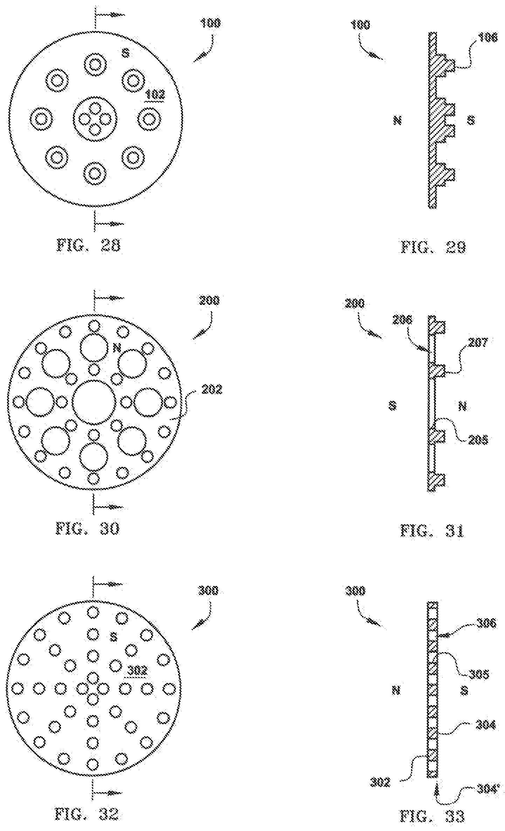

Thus, Figures 29 through 33 illustrate just one example in keeping with the present invention in which more than two layers are aligned. As illustrated in Figure 32, the second member, in addition to having receiving zones **206** for receiving protrusions **106** from the first member **100,** may also have protrusions **207.** These protrusions **207** may then be received by receiving zones **306** of a third member **300.** Indeed, or alternatively, the protrusions **106** of the first member **100** may be received by receiving zones **306** of a third member **300** as illustrated in Figure 33.

As a result, magnetic repulsion of faces **102** and **202** forces the first member **100** and second member **200** apart and magnetic repulsion of faces 202 and **302** force the second member **200** and third member **300** apart, and so on (not shown). Combined, the multi-layered magnetic device exhibits a dynamic plurality of magnetic flux lines at an application surface **304'** when the layers are pressed together and then released, as discussed above.

## Claims

1. A device for therapeutic use on a mammal, comprising:
- a first member (100) having a plurality of protrusions (106), and
- a second member (200) defining an application surface and having a plurality of receiving zones (206) for aligning with the plurality of protrusions (106) of the first member (100),
- wherein at least a portion of the first member (100) has a first magnetic pattern (108), wherein at least a portion of the second member (200) has a second magnetic pattern (208), wherein the protrusions (106) are aligned with the receiving zones (206), wherein at least one region of the first member (100) faces at least one region of the second member (200) forcing together magnetic patterns (108, 208) of like polarity on the first member facing region and the second member facing region, thereby repulsing one another and producing a plurality of magnetic flux lines at the application surface that vary in time as the distance between the first and second members (100, 200) varies in time resulting in a therapeutic effect on the mammal being treated thereby,
- wherein the first magnetic pattern (108) extends across at least one protrusion of the plurality of protrusions (106) so that the first magnetic pattern (108) on a face of the at least one protrusion (106) contributes to a magnetic pattern of an application surface of the device.

2. A device as in claim 1 wherein there are two or more protrusions (106) and two or more receiving zones (206) configured so that when the protrusions (106) are received in the receiving zones (206), the first member (100) is fixed relative to the second member (200) in a plane generally parallel to a plane defined by the second member (200).

3. A device as in one of claims 1 or 2 wherein the protrusions (106) loosely fit within the receiving zones (206) such that the receiving zones (206) can move along the protruding dimension of the protrusions (106).

4. A device as in one of claims 1 to 3 wherein the protrusions (106) loosely fit within the receiving zones (206) such that the protrusions (106) can move in the receiving zones (206).

5. A device as in claim 4 wherein the movement of the protrusions (106) in the receiving zones (206) is a direction generally perpendicular to the second member (200).

6. A method for forming a device for use on a mammal, comprising:
- placing a first magnetized sheet (100) having a first magnetic pattern (108) beside a second magnetized sheet (200) having a second magnetic pattern (208),
- aligning a plurality of keying features (106) on the first sheet (100) with a plurality of receiving features (206) on the second sheet (200), the keying features (106) being adapted to being received in the receiving features (206),
- causing thereby at least one region of the first magnetic pattern (108) to face at least one region of the second magnetic pattern (208) of like polarity, and
- producing a therapeutically effective plurality of magnetic flux lines at an application surface of the device, wherein the plurality of magnetic flux lines varies as a user varies the distance between the first and second sheets (100, 200) during use,
- wherein the first magnetic pattern (108) extends across at least one of the keying features (106) so that the magnetic pattern on the face of the keying feature (106) contributes to the magnetic pattern of the application surface.

7. A method as in claim 6 wherein there are two or more keying features (106) and two or more receiving features (206) so that when the keying features (106) are received in the receiving features (206), the first sheet (100) cannot move relative to the second sheet (200) in a plane generally parallel to a plane defined by the second sheet (200).

8. A method as in one of claims 6 or 7 wherein the keying features (106) loosely fit within the receiving features (206) such that the receiving features (206) can move generally perpendicular to a plane defined by the application surface.

9. A method as in one of claims 6 to 8 wherein the keying features (106) loosely fit within the receiving features (206) such that the keying features (106) can move in the receiving features (206).

## Patentansprüche

1. Vorrichtung zur therapeutischen Verwendung an einem Säugetier, aufweisend:
- ein erstes Element (100), das mehrere Vorsprünge (106) aufweist, und
- ein zweites Element (200), das eine Anwendungsfläche definiert und mehrere Aufnahmebereiche (206) für eine Ausrichtung mit den mehreren Vorsprüngen (106) des ersten Elements (100) aufweist,
- wobei mindestens ein Teil des ersten Elements (100) ein erstes magnetisches Muster (108) aufweist, wobei mindestens ein Teil des zweiten Elements (200) ein zweites magnetisches Muster (208) aufweist, wobei die Vorsprünge (106) mit den Aufnahmebereichen (206) ausgerichtet sind, wobei mindestens ein Bereich des ersten Elements (100) mindestens einem Bereich des zweiten Elements (200) zugewandt ist, wobei magnetische Muster (108, 208) gleicher Polarität an dem dem ersten Element zugewandten Bereich und dem dem zweiten Element zugewandten Bereich zusammengedrückt werden, wodurch diese sich voneinander abstoßen und eine Vielzahl magnetischer Flusslinien an der Anwendungsfläche erzeugen, die sich mit der Zeit verändern, wenn sich der Abstand zwischen den ersten und zweiten Elementen (100, 200) mit der Zeit verändert, was in einem therapeutischen Effekt an dem dadurch behandelten Säugetier resultiert,
- wobei sich das erste magnetische Muster (108) über mindestens einen Vorsprung der mehreren Vorsprünge (106) erstreckt, so dass das erste magnetische Muster (108) an einer Fläche des mindestens einen Vorsprungs (106) zu einem magnetischen Muster einer Anwendungsfläche der Vorrichtung beiträgt.

2. Vorrichtung nach Anspruch 1, wobei zwei oder mehr Vorsprünge (106) und zwei oder mehr Aufnahmebereiche (206) vorgesehen sind, die derart ausgebildet sind, dass, wenn die Vorsprünge (106) in den Aufnahmebereichen (206) aufgenommen sind, das erste Element (100) relativ zu dem zweiten Element (200) in einer Ebene befestigt ist, die im Allgemeinen parallel zu einer Ebene liegt, die von dem zweiten Element (200) definiert wird.

3. Vorrichtung nach einem der Ansprüche 1 oder 2, wobei die Vorsprünge (106) lose derart in die Aufnahmebereiche (206) passen, dass sich die Aufnahmebereiche (206) entlang der vorstehenden Abmessung der Vorsprünge (106) bewegen können.

4. Vorrichtung nach einem der Ansprüche 1 bis 3, wobei die Vorsprünge (106) lose derart in die Aufnahmebereiche (206) passen, dass sich die Vorsprünge (106) in den Aufnahmebereichen (206) bewegen können.

5. Vorrichtung nach Anspruch 4, wobei die Bewegung der Vorsprünge (106) in den Aufnahmebereichen (206) in einer Richtung im Allgemeinen senkrecht zu dem zweiten Element (200) verläuft.

6. Verfahren zum Bilden einer Vorrichtung zur Verwendung an einem Säugetier, umfassend:
- Anordnen einer ersten magnetisierten Fläche (100), die ein erstes magnetisches Muster (108) aufweist, neben einer zweiten magnetisierten Fläche (200), die ein zweites magnetisches Muster (208) aufweist,
- Ausrichten mehrerer Schlüsseleinrichtungen (106) an der ersten Fläche (100) mit mehreren Aufnahmeeinrichtungen (206) an der zweiten Fläche (200), wobei die Schlüsseleinrichtungen (106) dafür ausgelegt sind, in den Aufnahmeeinrichtungen (206) aufgenommen zu werden,
- dadurch bewirken, dass mindestens ein Bereich des ersten magnetischen Musters (108) mindestens einem Bereich des zweiten magnetischen Musters (208) gleicher Polarität zugewandt ist, und
- Erzeugen einer therapeutisch wirksamen Vielzahl magnetischer Flusslinien an einer Anwendungsfläche der Vorrichtung, wobei sich die Vielzahl der magnetischen Flusslinien verändert, wenn ein Nutzer den Abstand zwischen den ersten und zweiten Flächen (100, 200) während der Benutzung verändert,
- wobei sich das erste magnetische Muster (108) über mindestens eine der Schlüsseleinrichtungen (106) derart erstreckt, dass das magnetische Muster an der Fläche der Schlüsseleinrichtung (106) zu dem magnetischen Muster der Anwendungsfläche beiträgt.

7. Verfahren nach Anspruch 6, wobei zwei oder mehr Schüsseleinrichtungen (106) und zwei oder mehr Aufnahmeeinrichtungen (206) vorgesehen sind, so dass, wenn die Schlüsseleinrichtungen (106) in den Aufnahmeeinrichtungen (206) aufgenommen sind, die erste Fläche (100) sich nicht relativ zu der zweiten Fläche (200) in einer Ebene im Allgemeinen parallel zu einer Ebene bewegen kann, die von der zweiten Fläche (200) definiert wird.

8. Verfahren nach einem der Ansprüche 6 oder 7, wobei die Schlüsseleinrichtungen (106) lose derart in die Aufnahmeeinrichtungen (206) hineinpassen, dass sich die Aufnahmeeinrichtungen (206) im Allgemeinen senkrecht zu einer Ebene bewegen können, die von der Anwendungsfläche definiert wird.

9. Verfahren nach einem der Ansprüche 6 bis 8, wobei die Schlüsseleinrichtungen (106) lose derart in die Aufnahmeeinrichtungen (206) hineinpassen, dass sich die Schlüsseleinrichtungen (106) in den Aufnahmeeinrichtungen (206) bewegen können.

## Revendications

1. Dispositif destiné à un usage thérapeutique sur un mammifère, qui comprend :
- un premier élément (100) qui possède une pluralité de saillies (106), et
- un second élément (200) qui définit une surface d'application et qui possède une pluralité de zones de réception (206) destinées à s'aligner avec la pluralité de saillies (106) du premier élément (100),
- dans lequel au moins une partie du premier élément (100) possède un premier motif magnétique (108), dans lequel au moins une partie du second élément (200) possède un second motif magnétique (208), dans lequel les saillies (106) sont alignées avec les zones de réception (206), dans lequel au moins une zone du premier élément (100) fait face à au moins une zone du second élément (200) en forçant ensemble les motifs magnétiques (108, 208) de la même polarité sur la zone tournée vers le premier élément et la zone tournée vers le second élément, afin qu'ils se repoussent, et afin de produire une pluralité de lignes de flux magnétique au niveau de la surface d'application, qui varient dans le temps lorsque la distance entre le premier et le second éléments (100, 200) varie dans le temps, ce qui entraîne un effet thérapeutique sur le mammifère traité,
- dans lequel le premier motif magnétique (108) s'étend sur au moins une saillie de la pluralité de saillies (106) de sorte que le premier motif magnétique (108) situé une face d'au moins une saillie (106) contribue à un motif magnétique d'une surface d'application du dispositif.

2. Dispositif selon la revendication 1, dans lequel se trouvent deux saillies ou plus (106) et deux zones de réception ou plus (206) configurées de sorte que, lorsque les saillies (106) sont reçues dans les zones de réception (206), le premier élément (100) soit fixe par rapport au second élément (200) sur un plan généralement parallèle à un plan défini par le second élément (200).

3. Dispositif selon l'une des revendications 1 ou 2, dans lequel les saillies (106) se placent librement dans les zones de réception (206) de sorte que les zones de réception (206) puissent se déplacer le long de la dimension de saillie des saillies (106).

4. Dispositif selon l'une des revendications 1 à 3, dans lequel les saillies (106) se placent librement dans les zones de réception (206) de sorte que les saillies (106) puissent se déplacer dans les zones de réception (206).

5. Dispositif selon la revendication 4, dans lequel le mouvement des saillies (106) dans les zones de réception (206) est une direction généralement perpendiculaire au second élément (200).

6. Procédé de formation d'un dispositif destiné à être utilisé sur un mammifère, qui comprend :
- le placement d'une première feuille magnétisée (100) qui possède un premier motif magnétique (108) à côté d'une seconde feuille magnétisée (200) qui possède un second motif magnétique (208),
- l'alignement d'une pluralité de dispositifs de verrouillage (106) de la première feuille (100) avec une pluralité de dispositifs de verrouillage (206) de la seconde feuille (200), les dispositifs de verrouillage (106) étant adaptés pour être reçus dans les dispositifs de réception (206),
- le fait qu'au moins une zone du premier motif magnétique (108) soit tournée vers au moins une zone du second motif magnétique (208) de la même polarité, et
- la production d'une pluralité de lignes de flux magnétique thérapeutiquement efficaces au niveau d'une surface d'application du dispositif, la pluralité de lignes de flux magnétique variant lorsqu'un utilisateur fait varier la distance entre la première et la seconde feuilles (100, 200) pendant l'utilisation,
- dans lequel le premier motif magnétique (108) s'étend sur au moins l'un des dispositifs de verrouillage (106) de sorte que le motif magnétique situé sur la face du dispositif de verrouillage (106) contribue au motif magnétique de la surface d'application.

7. Procédé selon la revendication 6, dans lequel se trouvent deux dispositifs de verrouillage ou plus (106) et deux dispositifs de réception ou plus (206) de sorte que, lorsque les dispositifs de verrouillage (106) sont reçus dans les dispositifs de réception (206), la première feuille (100) ne puisse pas se déplacer par rapport à la seconde feuille (200) sur un plan généralement parallèle à un plan défini par la seconde feuille (200).

8. Procédé selon l'une des revendications 6 ou 7, dans lequel les dispositifs de verrouillage (106) se placent librement dans les dispositifs de réception (206) de sorte que les dispositifs de réception (206) puissent se déplacer de manière généralement perpendiculaire à un plan défini par la surface d'application.

9. Procédé selon l'une des revendications 6 à 8, dans lequel les dispositifs de verrouillage (106) se placent librement dans les dispositifs de réception (206) de sorte que les dispositifs de verrouillage (106) puissent se déplacer dans les dispositifs de réception (206).
